(19) Europäisches Patentamt
European Patent Office
Office européen des brevets

(11) Veröffentlichungsnummer: **0 313 984 A1**

(12) **EUROPÄISCHE PATENTANMELDUNG**

(21) Anmeldenummer: **88117455.1**

(22) Anmeldetag: **20.10.88**

(51) Int. Cl.⁴: **C07D 417/06 , A61K 31/425**

Die Bezeichnung der Erfindung wurde geändert (Richtlinien für die Prüfung im EPA, A-III, 7.3).

(30) Priorität: **30.10.87 DE 3736783**

(43) Veröffentlichungstag der Anmeldung: **03.05.89 Patentblatt 89/18**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Krämer, Wolfgang, Dr.**
**Rosenkranz 25**
**D-5093 Burscheid 2(DE)**
Erfinder: **Regel, Erik**
**Untere Bergerheide 26**
**D-5600 Wuppertal 1(DE)**
Erfinder: **Büchel, Karl Heinz, Prof. Dr.**
**Dabringhauser Strasse 42**
**D-5093 Burscheid(DE)**
Erfinder: **Plempel, Manfred, Dr.**
**Zwengenberger Strasse 3 c**
**D-5657 Haan(DE)**

(54) **Substituierte Azolylmethylcarbinole und diese enthaltende Arzneimittel.**

(57) Die Erfindung betrifft substituierte Azolylmethylcarbinole der Formel

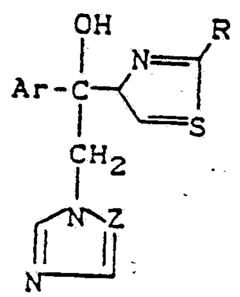

(I)

in welcher
Ar für gegebenenfalls substituiertes Aryl steht,
R für Alkyl, Alkenyl, Alkinyl, Alkoxyalkyl, für jeweils gegebenenfalls substituiertes Aralkyl, Aryloxyalkyl oder Aryl oder für einen Rest

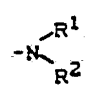

steht und
Z für Stickstoff oder für eine CH-Gruppe steht, wobei
$R^1$ für Wasserstoff, Alkyl, Alkenyl, Alkinyl oder Cycloalkyl steht und

EP 0 313 984 A1

$R^2$ für Wasserstoff, Alkyl, Alkoxyalkyl, Alkenyl, Alkinyl, Cycloalkyl, Cycloalkylalkyl, Arylalkyl oder für gegebenenfalls substituiertes Aryl steht oder
$R^1$ und $R^2$ gemeinsam mit dem Stickstoffatom, an welches sie gebunden sind, für einen gegebenenfalls substituierten Heterocyclus stehen, der gegebenenfalls weitere Heteroatome enthalten kann,
sowie diese Azolylmethylcarbinole enthaltende Arzneimittel zur Behandlung von Krankheiten, insbesondere Mykosen.

## Verwendung neuer Azolylmethylcarbinole zur Behandlung von Krankheiten

Die Erfindung betrifft die Verwendung von neuen substituierten Azolylmethylcarbinolen zur Behandlung von Krankheiten, insbesondere von Mykosen.

Es ist bekannt, daß bestimmte Azolylmethylcarbinole, wie beispielsweise das 2-Phenyl-1,3-bis-(1,2,4-triazol-1-yl)-propan-2-ol oder das 2-(4-Fluorphenyl)-3-methyl-1-imidazol-1-yl-3-(1,2,4-triazol-1-yl)-butan-2-ol antimykotische Eigenschaften besitzen (vgl. z.B. EP 44 605 sowie EP 120 276).

Die Wirksamkeit dieser vorbekannten Verbindungen ist jedoch nicht in allen Indikationen völlig zufriedenstellend.

Es wurde gefunden, daß die neuen substituierten Azolylmethylcarbinole der allgemeinen Formel (I),

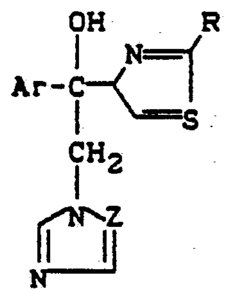

(I)

in welcher

Ar für gegebenenfalls substituiertes Aryl steht,

R für Alkyl, Alkenyl, Alkinyl, Alkoxyalkyl, für jeweils gegebenenfalls substituiertes Aralkyl, Aryloxyalkyl oder Aryl oder für einen Rest

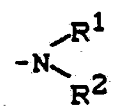

steht und

Z für Stickstoff oder für eine CH-Gruppe steht, wobei

$R^1$ für Wasserstoff, Alkyl, Alkenyl, Alkinyl oder Cycloalkyl steht und

$R^2$ für Wasserstoff, Alkyl, Alkoxyalkyl, Alkenyl, Alkinyl, Cycloalkyl, Cycloalkylalkyl, Arylalkyl oder für gegebenenfalls substituiertes Aryl steht oder

$R^1$ und $R^2$ gemeinsam mit dem Stickstoffatom, an welches sie gebunden sind, für einen gegebenenfalls substituierten Heterocyclus stehen, der gegebenenfalls weitere Heteroatome enthalten kann,

sowie deren physiologisch verträgliche Säureadditionssalze gute antimikrobielle, insbesondere gute antimykotische Eigenschaften besitzen.

Die Verbindungen der Formel (I) können als optische Isomere oder Isomerengemische unterschiedlicher Zusammensetzung vorliegen. Sowohl die reinen Isomeren als auch die Isomerengemische werden erfindungegemäß beansprucht.

Überraschenderweise zeigen die neuen erfindungegemäß verwendbaren substituierten Azolylmethylcarbinole der allgemeinen Formel (I) in bestimmten Indikationen eine deutlich bessere antimykotische Wirksamkeit als die aus dem Stand der Technik bekannten Azolylmethylcarbinole, wie beispielsweise das 2-Phenyl-1,3-bis-(1,2,4-triazol-1-yl)-propan-2-ol oder das 2-(4-Fluorphenyl)-3-methyl-1-imidazol-1-yl-3-(1,2,4-triazol-1-yl)-butan-2-ol, welche chemisch und wirkungsmäßig neheliegende Verbindungen sind.

Die erfindungsgemäß verwendbaren substituierten Azolylmethylcarbinole sind durch die Formel (I) allgemein definiert. Bevorzugt erfindungsgemäß verwendbar sind Verbindungen der Formel (I), bei welchen

Ar für gegebenenfalls einfach oder mehrfach, gleich oder verschieden substituiertes Phenyl steht, wobei als Substituenten infrage kommen: Halogen, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen

Halogenatomen, insbesondere Fluor, Chlor oder Brom.
R für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen oder Alkoxyalkyl mit 1 bis 6 Kohlenstoffatomen in den einzelnen Alkylteilen, für jeweils gegebenenfalls im Arylteil einfach oder mehrfach, gleich oder verschieden substituiertes, gegebenenfalls im Alkylteil geradkettiges oder verzweigtes Aralkyl, Aryloxyalkyl oder Aryl mit gegebenenfalls 1 bis 4 Kohlenstoffatomen im Alkylteil und jeweils 6 bis 10 Kohlenstoffatomen im Arylteil steht, wobei als Substituenten im Arylteil jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor oder Brom, Phenyl sowie ein gegebenenfalls einfach oder mehrfach, gleich oder verschieden durch jeweils geradkettiges oder verzweigtes Alkyl, Alkoxycarbonyl oder Alkylcarbamoyl mit jeweils 1 bis 4 Kohlenstoffatomen im Alkylteil substituierter N-verknüpfter 5-Ring- oder 6-Ring-Heterocyclus, der gegebenenfalls weitere Heteroatome wie insbesondere Stickstoff, Sauerstoff oder Schwefel enthalten kann;
außerdem für einen Rest

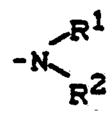

steht und
Z für Stickstoff oder für eine CH-Gruppe steht, wobei
$R^1$ für Wasserstoff, für jeweils geradkettiges oder verzweigtes Alkyl, Alkenyl oder Alkinyl mit jeweils bis zu 6 Kohlenstoffatomen oder für Cycloaklyl mit 3 bis 7 Kohlenstoffatomen steht und
$R^2$ für Wasserstoff, für jeweils geradkettiges oder verzweigtes Alkyl mit 1-12 Kohlenstoffatomen, Alkoxyalkyl mit 1-4 Kohlenstoffatomen in den einzelnen Alkylteilen, Alkenyl oder Alkinyl mit jeweils bis zu 6 Kohlenstoffatomen, für Cycloalkyl mit 3-7 Kohlenstoffatomen, Cycloalkylalkyl mit 3-7 Kohlentstoffatomen im Cycloalkylteil und 1-4 Kohlenstoffatomen im Alkylteil, Arylalkyl mit 1-4 Kohlenstoffatomen im Alkylteil oder für gegegebenenfalls einfach oder mehrfach, gleich oder verschieden substituiertes Phenyl steht, wobei als Substituenten in Frage kommen: Halogen, insbesondere Fluor, Chlor oder Brom, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor oder Brom oder
$R^1$ und $R^2$ gemeinsam mit dem Stickstoffatom, an welches sie gebunden sind, für einen gegebenenfalls einfach oder mehrfach, gleich oder verschieden substituierten, gesättigten Heterocyclus mit 5 bis 7 Ringgliedern stehen, der gegebenenfalls ein weiteres Heteroatom, insbesondere Stickstoff, Sauerstoff oder Schwefel enthalten kann, wobei als Substituenten jeweils infrage kommen: jeweils geradkettiges oder verzweigtes Alkyl, Hydroxyalkyl, Alkanoyl oder Alkanoyloxyalkyl mit jeweils 1 bis 4 Kohlenstoffatomen in den einzelnen Alkylteilen.

Besonders bevorzugt erfindungsgemäß verwendbar sind Verbindungen der Formel (I), bei welchen
Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio,
R für Methyl, Ethyl, n- oder i-Propyl, n- i-, s-oder t-Butyl, Allyl, n- oder i-Butenyl, Propargyl, Methoxymethyl, Methoxyethyl, Ethoxymethyl, Ethoxyethyl, n- oder i-Propoxymethyl, n- oder i-Propoxyethyl oder für jeweils gegebenenfalls im Phenylteil ein- bis dreifach, gleich oder verschieden substituiertes Benzyl, Phenylethyl, Phenoxymethyl oder Phenyl steht, wobei als Substituenten jeweils infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Phenyl sowie jeweils gegebenenfalls ein- bis dreifach, gleich oder verschieden durch Methyl, Ethyl, Methoxycarbonyl, Ethoxycarbonyl, Methylcarbamoyl oder Ethylcarbamoyl substituiertes Triazolyl, Imidazolyl, Pyrazolyl, Pyrrolidinyl, Piperidinyl, Morpholinyl oder Piperazinyl;
außerdem für einen Rest

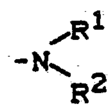

steht und
Z für Stickstoff oder für eine CH-Gruppe steht, wobei
$R^1$ für Wasserstoff, Methyl, Ethyl, n- oder i-Propyl, n-, i-, s- oder t-Butyl, Allyl, n- oder i-Butenyl, Propargyl, n- oder i-Butinyl, Cyclopentyl oder Cyclohexyl steht und
$R^2$ für Wasserstoff, Methyl, Ethyl, n- oder i-Propyl, n-, i-, s- oder t-Butyl, Allyl, n- oder i-Butenyl, Propargyl, n- oder i-Butinyl, Cyclopentyl, Cyclohexyl oder für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio oder
$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

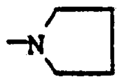 ; 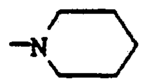 ; 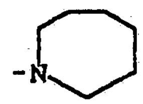 ; 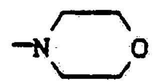 ;

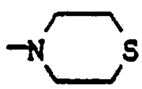 oder 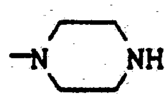

stehen, wobei
als Substituenten jeweils infrage kommen: Methyl, Ethyl, Hydroxymethyl, Acetyl, Propionyl oder Acetoxymethyl.

Ganz besonders bevorzugt sind Verbindungen der Formel (I), bei welchen
Ar für gegebenenfalls ein- bis zweifach, gleich oder verschieden durch Fluor, Chlor, Brom, Methyl, Methoxy, Trifluormethyl, Trifluormethoxy oder Trifluormethylthio substituiertes Phenyl steht,
R für Methyl, Ethyl, n- oder i-Propyl, Allyl, Propargyl, Methoxymethyl, für jeweils gegebenenfalls ein- bis zweifach, gleich oder verschieden substituiertes Benzyl, Phenoxymethyl oder Phenyl steht, wobei als Phenylsubstituenten jeweils infrage kommen: Fluor, Chlor, Brom, Methyl, Methoxy, Methylthio, Trifluormethyl, Trifluormethoxy, Trifluormethylthio, Phenyl sowie jeweils gegebenenfalls einfach oder zweifach, gleich oder verschieden durch Methyl, Methoxycarbonyl, Ethoxycarbonyl oder Methylcarbonyl substituiertes Triazolyl, Imidazolyl, Pyrazolyl, Piperidinyl, Morpholinyl oder Piperazinyl;
außerdem für einen Rest

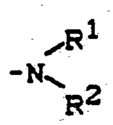

steht und
Z für Stickstoff oder für eine CH-Gruppe steht, wobei
$R^1$ für Wasserstoff, Methyl, Ethyl, n- oder i-Propyl oder Cyclohexyl steht,
$R^2$ für Wasserstoff, Methyl, Ethyl, n- oder i-Propyl, Cyclohexyl oder für gegebenenfalls einfach oder zweifach, gleich oder verschieden durch Fluor, Chlor, Brom, Methyl, Methoxy, Trifluormethyl, Trifluormethoxy oder Trifluormethylthio substituiertes Phenyl steht oder
$R^1$ und $R^2$ gemeinsam mit dem Stickstoffatom, an welches sie gebunden sind für einen gegebenenfalls ein- bis dreifach durch Methyl substituierten Heterocyclus der Formel

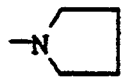 ; 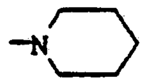 oder 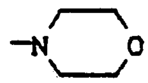

stehen oder für einen gegebenenfalls am Stickstoff durch Methyl, Ethyl, Acetyl oder Propionyl substituierten Heterocyclus der Formel

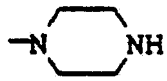

stehen.

Bevorzugte erfindungsgemäß verwendbare Verbindungen sind auch Additionsprodukte aus Säuren und denjenigen substituierten Azolylmethylcarbinolen der Formel (I), in denen die Substituenten Ar, R und Z die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Im einzelnen seien außer den bei den Herstellungsbeispielen genannten Verbindungen die folgenden substituierten Azolylmethylcarbinole der allgemeinen Formel (I) genannt:

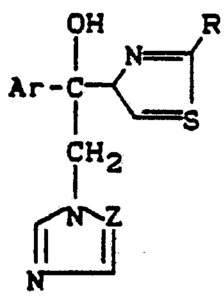

(I)

| Ar | R | Z |
|---|---|---|
| 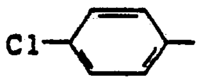 | 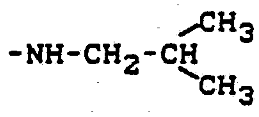 | N |
| 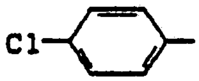 | $-NH-(CH_2)_{11}-CH_3$ | N |
| 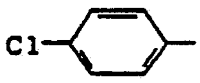 | $-NH-(CH_2)_5-CH_3$ | N |
| 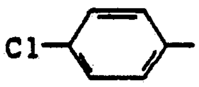 | $-NH-CH_2-$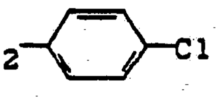 | N |
| 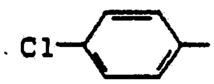 | $-NH-(CH_2)_2-CH_3$ | N |
| 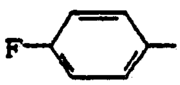 | $-NH-C_2H_5$ | N |
| 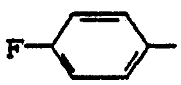 | $-NH-CH_2-CH_2-$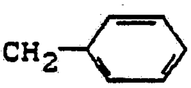 | N |
| 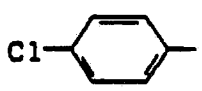 | $-NH-(CH_2)_3-N(CH_3)_2$ | N |

| Ar | R | Z |
|---|---|---|
| 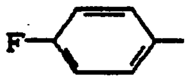 | 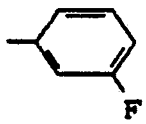 | N |
| 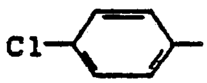 | 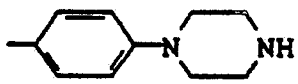 | N |
| 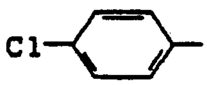 | 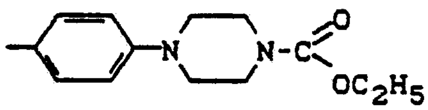 | N |
| 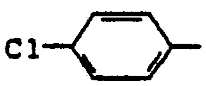 | 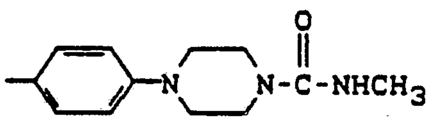 | N |
| 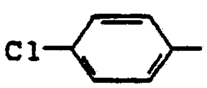 | 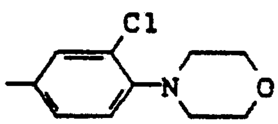 | N |
| 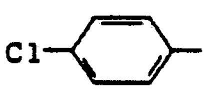 | 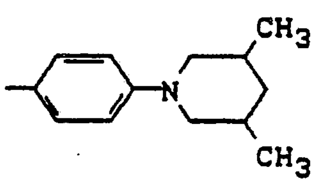 | N |
| 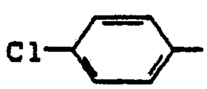 | 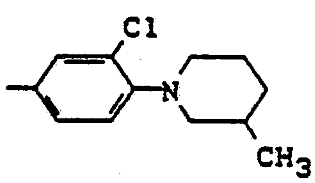 | N |

| Ar | R | Z |
|---|---|---|
| 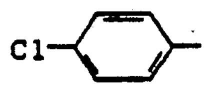 | 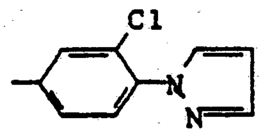 | N |
| 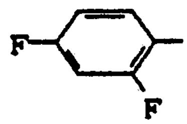 | $-(CH_2)_3-CH_3$ | N |
| 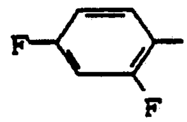 | $-(CH_2)_2-CH_3$ | N |

Die erfindungemäß verwendbaren substituierten Azolylmethylcarbinole der Formel (I) sind noch nicht bekannt. Sie sind jedoch Gegenstand einer eigenen parallelen Patentanmeldung und können nach dem dort beschriebenen Verfahren erhalten werden, indem man substituierte 2-Bromethanole der Formel (II),

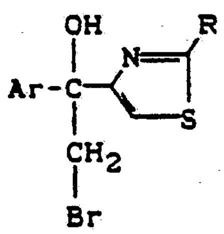 (II)

in welcher
Ar und R die oben angegebene Bedeutung haben,
mit Azolen der Formel (III),

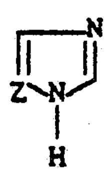 (III)

in welcher
Z die oben angegebene Bedeutung hat,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt und gegebenenfalls anschließend eine Säure addiert.

Verwendet man beispielsweise 1-(4-Chlorphenyl)-1-(2-methylthiazol-4-yl)-2-bromethanol und 1,2,4-Triazol als Ausgangsstoffe, so läßt sich der Reaktionsablauf des Herstellungsverfahrens durch das folgende Formelschema darstellen:

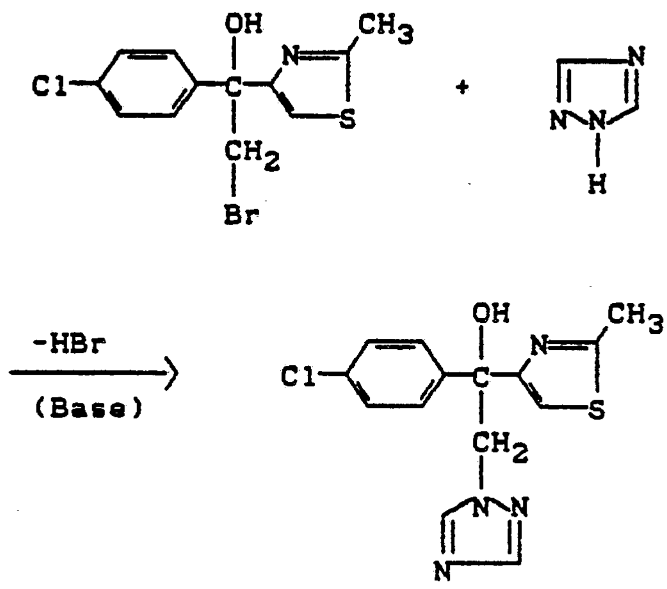


Die zur Durchführung des Herstellungsverfahrens als Ausgangsstoffe benötigten substituierten 2-Bromethanole sind durch die Formel (II) allgemein definiert. In dieser Formel (II) stehen Ar und R vorzugsweise für diejenigen Reste, die Bereits im Zusammenhang mit der Beschriebung der erfindungsgemäß verwendbaren Stoffe der Formel (I) als bevorzugt für diese Substituenten genannt wurden.

Die substituierten 2-Bromethanole der Formel (II) sind noch nicht bekannt. Man erhält sie, wenn man Dibromacyloine der Formel (IV),

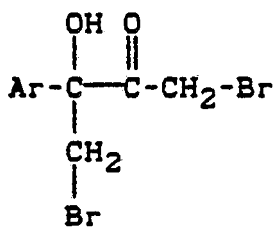 (IV)

in welcher
Ar die oben angegebene Bedeutung hat,
mit Thiocarbonsäureamiden der Formel (V),

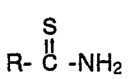 (V)

in welcher
R die oben angegebene Bedeutung hat,
zunächst in Gegenwart einer Base wie beispielsweise Natriumhydrogencarbonat sowie in Gegenwart eines Verdünnungsmittels, wie beispielsweise Ethanol kondensiert und anschließend in Gegenwart einer Säure wie beispielsweise p-Toluolsulfonsäure sowie in Gegenwart eines Verdünnungsmittels wie beispielsweise Dichlormethan bei Temperaturen zwischen 0 °C und 60 °C cyclisiert (vgl. z.B. C.Ferri, "Reaktionen der organischen Synthese", S. 731, Thieme Verlag, Stuttgart 1978 und die Herstellungsbeispiele der vorliegenden Anmeldung).

Dibromacyloine der Formel (IV) sind bekannt (vgl. z.B. Helvetica Chim. Acta 29, 95-101 [1946]). Sie sind beispielsweise erhältlich, wenn man Dibromdiacetyl (vgl. Liebigs Ann, Chem. 249, 207 [1888]) mit Aromaten der Formel (VI),

Ar-H (VI)

in welcher
Ar die oben angegebenen Bedeutung hat,
in üblicher Art und Weise (Friedel-Crafts-Reaktion) in Gegenwart eines Katalysators wie beispielsweise Aluminium-III-chlorid bei Temperaturen zwischen 0 °C und 80 °C umsetzt (vgl. auch die Herstellungsbeispiele).

Thiocarbonsäureamide der Formel (V) und Aromaten der Formel (VI) sind allgemein bekannte Verbindungen der organischen Chemie.

Die zur Durchführung des Herstellungsverfahrens weiterhin als Ausgangsstoffe benötigten Azole sind durch die Formel (III) allgemein definiert. In dieser Formel (III) steht Z vorzugsweise für diejenigen Reste, die bereits im Zusammenhang mit der Beschriebung der erfindungsgemäß verwendbaren Stoffe der Formel (I) als bevorzugt für diesen Substituenten genannt wurden.

Die Azole der Formel (III) sind ebenfalls allgemein bekannte Verbindungen der organischen Chemie.

Als Verdünnungsmittel zur Durchführung des Herstellungsverfahrens kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Sulfoxide, wie Dimethylsulfoxid oder Alkohole wie Methanol, Ethanol, Propanol oder Butanol.

Das Herstellungsverfahren wird vorzugsweise in Gegenwart eines geeigneten Reaktionshilfsmittel durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das Herstellungsverfahren kann gegebenenfalls auch in einem Zweiphasensystem, wie beispielsweise Wasser/Toluol gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributylmethylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Die Reaktionstemperaturen können bei der Durchführung des Herstellungsverfahrens in einem größeren Bereich variiert werden. Im allgemeinen arbeitet man bei Temperaturen zwischen 20 °C und 200 °C, vorzugsweise bei Temperaturen zwischen 40 °C und 140 °C.

Zur Durchführung des Herstellungsverfahrens setzt man pro Mol an substituiertem 2-Bromethanol der Formel (II) im allgemeinen 1.0 bis 2.0 Mol, vorzugsweise 1.0 bis 1.5 Mol an Azol der Formel (III) und 1.0 bis 2.0 Mol, vorzugsweise 1.0 bis 1.5 Mol an Reaktionshilfsmittel ein. Die Reaktionsdurchführung, Aufarbeitung und Isolierung der Reaktionsprodukte erfolgt nach allgemein üblichen Methoden.

Zur Herstellung von physiologischverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin. Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampul len vorliegen, deren Wirkstoffgehalt einen Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder oder Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glucose, Mannit und Kieselsäure re, (b) Bindemittel, z.B. Carboxymethylcellulose, Alginate, Gelantine, Polyvinylpyrrolidon, (c) Feuchthaltemittel, z.B. Glycerin, (d) Sprengmittel z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Starke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethy lensorbit- und -sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben angeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise von 5 bis 150 mg/kg Körpergewicht je 24 Stunden, gegebenenfalls in Form mehrerer Einzelgaben zur Erzielung der gewünschten Ergebnisse zu verabreichen.

Bei oralen Applikationen werden die erfindungsgemäßen Wirkstoffe in Gesamtmengen von etwa 2,5 bis etwa 200, vorzugsweise von 5 bis 150 mg/kg Körpergewicht je 24 Stunden und bei parenteraler Applikation in Gesamtmengen von etwa 2,5 bis etwa 50, vorzugsweise von 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objektes, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführ te Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Biespiel 1

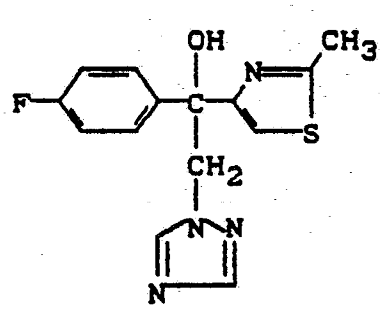

Zu einer Suspension zus 1,8 g (0.06 Mol) Natriumhydrid in 20 ml Dimethylformamid gibt man bei 10 °C tropfenweise unter Rühren eine Lösung von 4,1 g (0.06 Mol) Triazol in 30 ml Dimethylformamid und nach einer Stunde bei 0 °C ebenfalls tropfenweise unter Rühren eine Lösung von 12,6 g (0.04 Mol) 2-Brom-1-(4-fluorphenyl)-1-(2-methylthiazol-4-yl)-ethanol in 30 ml Dimethylformamid. Nach beendeter Zugabe rührt man eine Stunde bei 20 °C, zwei weitere Stunden bei 70 °C, zwei Stunden bei 90 °C und eine weitere Stunde bei 110 °C, Zur Aufarbeitung kühlt man die Reaktionsmischung ab, engt im Vakuum ein, nimmt den Rückstand in 300 ml Dichlormethan auf, wäscht zwei mal mit jeweils 200 ml Wasser, trocknet über Natriumsulfat, engt im Vakuum ein, chromatographiert über Kieselgel (Laufmittel:

Dichlormethan/Methanol 10:1) und kristallisiert das so erhältliche produkt .durch Verrühren mit 150 ml Diisopropylether.

Man erhält 3,5 g (29 % der Theorie) an 1-(4-Fluorphenyl)-1-(2-methylthiazol-4-yl)-2-(1,2,4-triazol-1-yl)-ethanol vom Schmelzpunkt 120 °C.

Herstellung der Ausgangsverbindung

Beispiel II-1

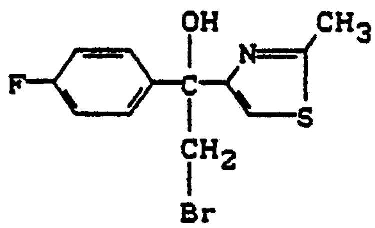

Zu 11,3 g (0.15 Mol) Thioacetamid in 100 ml Ethanol tropft man unter Rühren 51 g (0.15 Mol) 1,4-Dibrom-3-(4-fluorphenyl)-3-hydroxy-butan-2-on in 200 ml Ethanol, wobei die Temperatur der Reaktionsmischung auf 35 °C steigt, gibt anschließend 12,6 g (0.15 Mol) Natriumhydrogencarbonat zu, rührt 18 Stunden bei Raumtemperatur, engt dann im Vakuum ein, nimmt den Rückstand in 600 ml Dichlormethan auf, wäscht zweimal mit jeweils 200 ml Wasser, versetzt die Mischung mit 5,7 g (0.03 Mol) p-Toluolsulfonsäure und erhitzt für 24 Stunden über einem Wasserabscheider auf Rückflußtemperatur. Zur Aufarbeitung wäscht man zweimal mit jeweils 200 ml Wasser, trocknet über Natriumsulfat, engt im Vakuum ein und chromatographiert über Kieselgel (Laufmittel: Toluol).

Man erhält 28,5 (60 % der Theorie) an 2-Brom-1-(4-fluorphenyl)-1-(2-methylthiazol-4-yl)-ethanol als Öl vom Brechungsindex $n_D^{20}$ 1.5812.

Biespiel IV-1

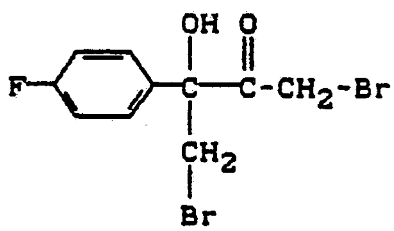

Zu 133 g (1 Mol) Aluminium-III-chlorid in 1 l Fluorbenzol gibt man innerhalb von 2 Stunden portionsweise 122 g (0.5 Mol) 1,4-Dibrombutan-2,3-dion, wobei die Temperatur der Reaktionsmischung auf 30 °C ansteigt. Nach beendeter Zugabe erwärmt man langsam auf 70 °C, rührt 2 Stunden bei dieser Temperatur, kühlt ab und gibt den Reaktionsansatz bei 0 °C in 3 l 0.2N Salzsäure. Zur Aufarbeitung wird die wässrige Phase abgetrennt, mit 300 ml Toluol extrahiert, die vereinigten organischen Phasen dreimal mit jeweils 500 ml Wasser gewaschen, über Natriumsulfat getrocknet, im Vakuum eingeengt und der Rückstand aus 75 ml Diisopropylether kristallisiert.

Man erhält 87,5 g (52 % der Theorie) an 1,4-Dibrom-3-(4-fluorphenyl)-3-hydroxybutan-2-on vom Schmelpunkt 90 °C.

In entsprechender Weise und gemäß den allgemeinen Angaben zur Herstellung erhält man die folgenden substituierten Azolylmethylcarbinole der allgemeinen Formel (I):

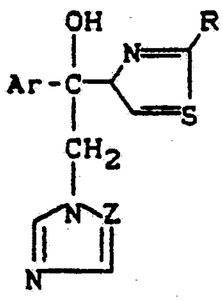

(I)

| Bsp. Nr. | Ar | R | Z | Schmelzpunkt /° C |
|---|---|---|---|---|
| 2 | 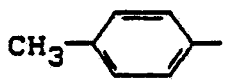 | 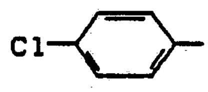 | N | 208 |
| 3 | 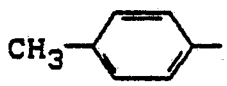 | 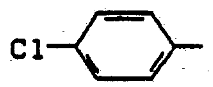 | CH | 228 |
| 4 | 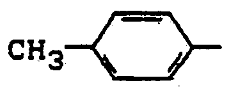 | 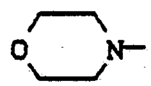 | N | 136 |
| 5 | 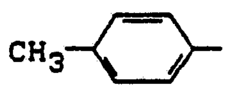 | 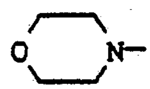 | CH | 156 |
| 6 | 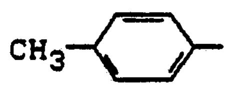 | 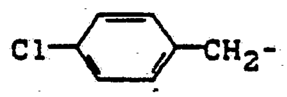 | N | 104 |

| Bsp. Nr. | Ar | R | Z | Schmelzpunkt /° C |
|---|---|---|---|---|
| 7 | 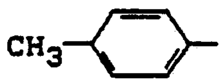 | 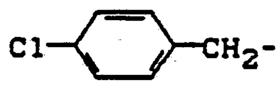 | CH | 122 |
| 8 | 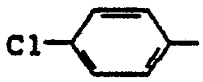 | 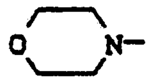 | N | 150 |
| 9 | 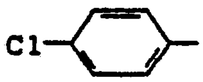 | 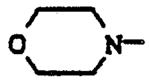 | CH | 188 |
| 10 | 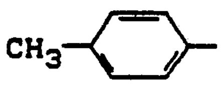 | 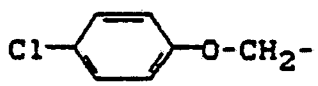 | N | 139 |
| 11 | 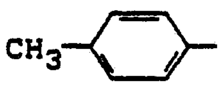 | 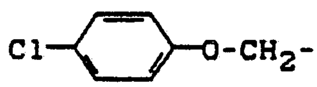 | CH | 138 |
| 12 | 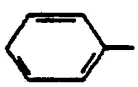 | 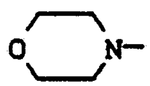 | CH | 160 |
| 13 | 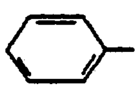 | 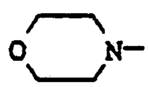 | N | 158 |
| 14 | 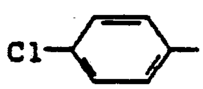 | 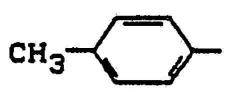 | CH | 208 |
| 15 | 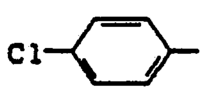 | 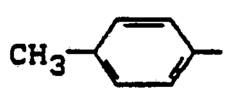 | N | 150 |

| Bsp. Nr. | Ar | R | Z | Schmelzpunkt /° C |
|---|---|---|---|---|
| 16 | 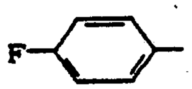 | 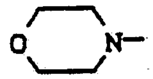 | CH | 142 |
| 17 | 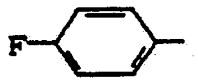 | 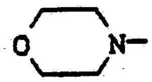 | N | 130 |
| 18 | 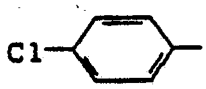 | 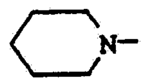 | CH | 174 |
| 19 | 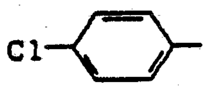 | 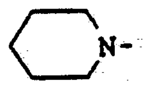 | N | 169 |
| 20 | 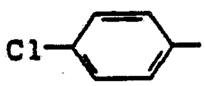 | 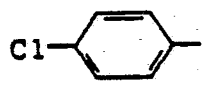 | CH | 230 |
| 21 | 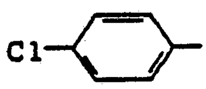 | 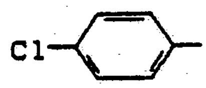 | N | 168 |
| 22 | 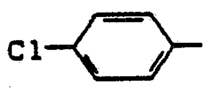 | 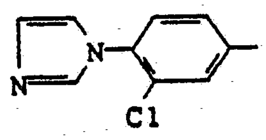 | CH | 200 |
| 23 | 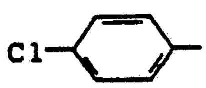 | 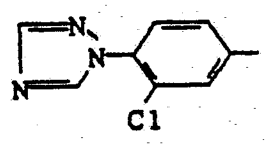 | N | 154 |

| Bsp. Nr. | Ar | R | Z | Schmelzpunkt /°C |
|---|---|---|---|---|
| 24 | 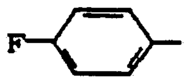 | $CH_3$ | CH | 196 |
| 25 | 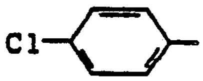 | 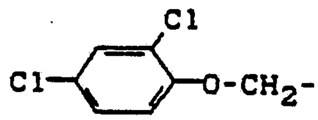 | CH | 136 |
| 26 | 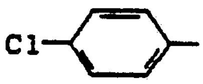 | 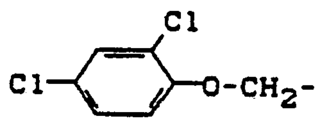 | N | 110 |
| 27 | 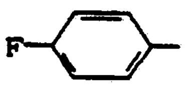 | 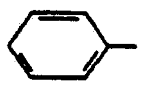 | CH | 149 |
| 28 | 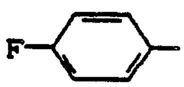 | 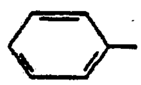 | N | 126 |
| 29 | 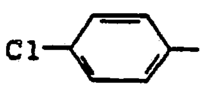 | 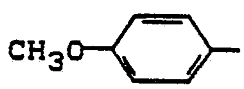 | CH | 205 |
| 30 | 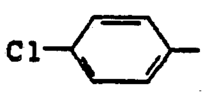 | 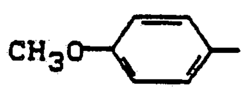 | N | 158 |
| 31 | 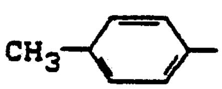 | 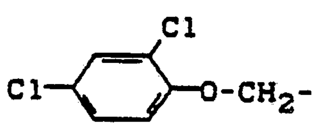 | N | 115 |

| Bsp. Nr. | Ar | R | Z | Schmelzpunkt /° C |
|---|---|---|---|---|
| 32 | 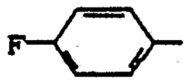 | $(CH_3)_2N-$ | CH | 148 |
| 33 | 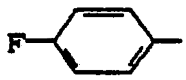 | $(CH_3)_2N-$ | N | 130 |
| 34 | 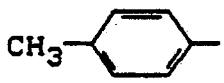 | 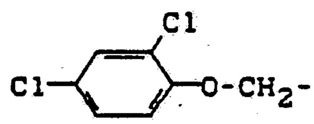 | CH | 96 |
| 35 | 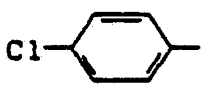 | 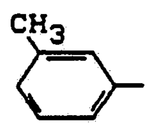 | CH | 182 |
| 36 | 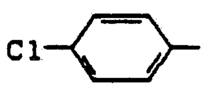 | 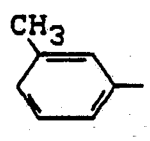 | N | 123 |
| 37 | 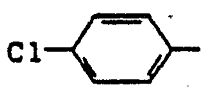 | $CH_3-NH-$ | N | 148 |
| 38 | 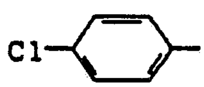 | $CH_3-NH-$ | CH | 176 |
| 39 | 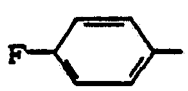 | 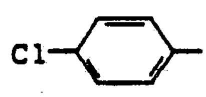 | CH | 225 |

| Bsp. Nr. | Ar | R | Z | Schmelzpunkt /° C |
|---|---|---|---|---|
| 40 | 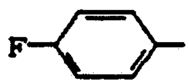 | 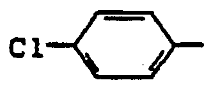 | N | 145 |
| 41 | 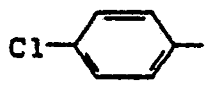 | 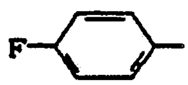 | N | 130 |
| 42 | 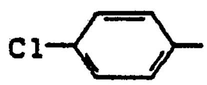 | 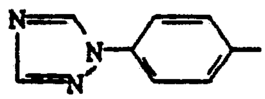 | N | 164 |
| 43 | 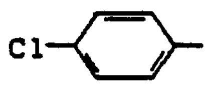 | 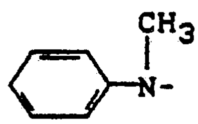 | N | 115 |
| 44 | 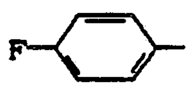 | 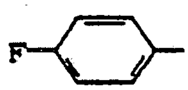 | N | 110 |
| 45 | 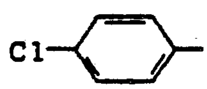 | 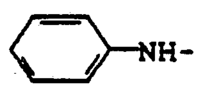 | N | 185 |
| 46 | 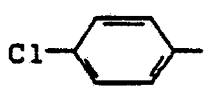 | $CH_2$=CH-$CH_2$-NH- | N | 126 |
| 47 | 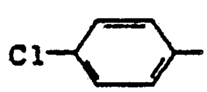 | 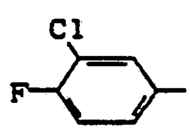 | N | 140 |

| Bsp. Nr. | Ar | R | Z | Schmelzpunkt /° C |
|---|---|---|---|---|
| 48 | 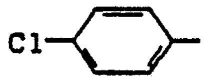 | 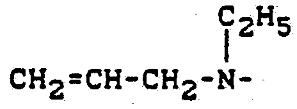 | N | 80 |
| 49 | 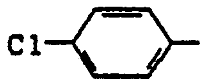 | $(C_2H_5)_2N-$ | N | 116 |
| 50 | 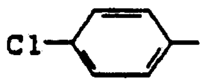 | 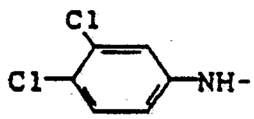 | N | 140 |
| 51 | 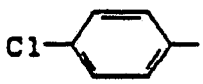 | $C_2H_5-NH-$ | N | 124 |
| 52 | 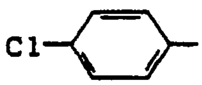 | 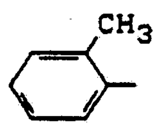 | N | 110 |
| 53 | 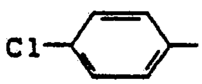 | 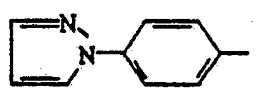 | N | 170 |
| 54 | 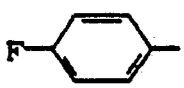 | 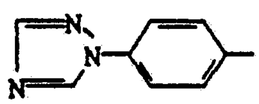 | N | 152 |
| 55 | 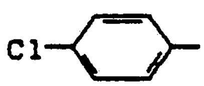 | 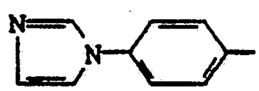 | N | 146 |

| Bsp. Nr. | Ar | R | Z | Schmelzpunkt /° C |
|---|---|---|---|---|
| 56 | 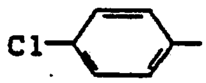 | 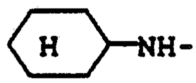 | N | 175 |
| 57 | 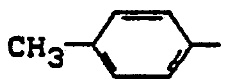 | $CH_3$ | N | 150 |
| 58 | 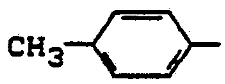 | 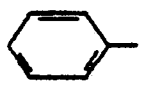 | N | 142 |
| 59 | 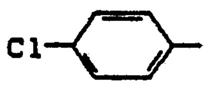 | 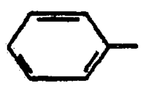 | N | 150 |
| 60 | 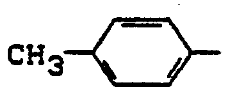 | $(CH_3)_2N-$ | N | 142 |
| 61 | 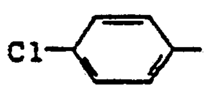 | $(CH_3)_2N-$ | N | 130 |
| 62 | 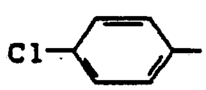 | 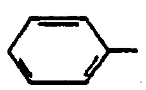 | CH | 171 |
| 63 | 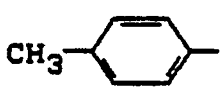 | $(CH_3)_2N-$ | CH | 163 |
| 64 | 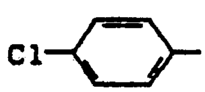 | $(CH_3)_2N-$ | CH | 177 |

| Bsp. Nr. | Ar | R | Z | Schmelzpunkt /°C |
|---|---|---|---|---|
| 65 | 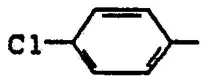 | $CH_3$ | N | 106 |
| 66 | 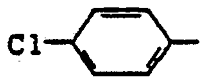 | $CH_3$ | CH | 182 |
| 67 | 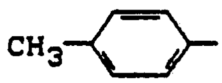 | $CH_3$ | CH | 160 |
| 68 | 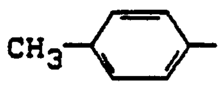 | 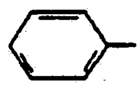 | CH | 165 |
| 69 | 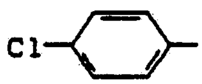 | 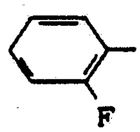 | N | 112 |
| 70 | 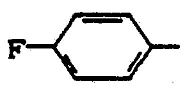 | 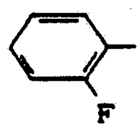 | N | 110 |
| 71 | 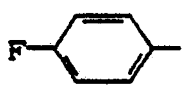 | 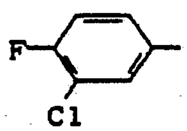 | N | 128 |
| 72 | 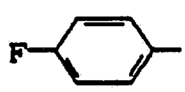 | 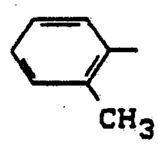 | N | 111 |

| Bsp. Nr. | Ar | R | Z | Schmelzpunkt /° C |
|---|---|---|---|---|
| 73 | 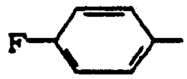 | 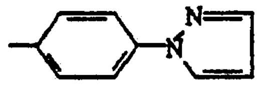 | N | 200 |
| 74 | 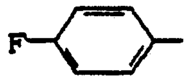 | 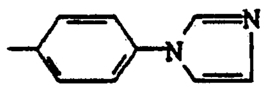 | N | 162 |
| 75 | 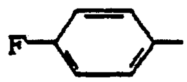 | 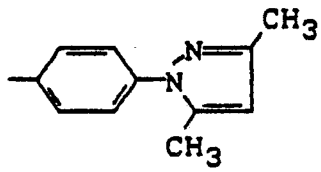 | N | 158 |
| 76 | 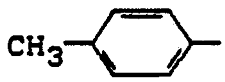 | 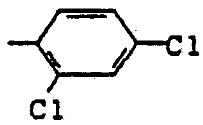 | N | 175 |
| 77 | 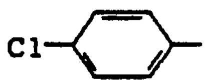 | -NH- 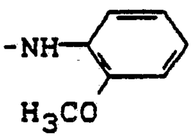 | N | 186 |
| 78 | 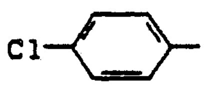 | $C_2H_5$ | N | 100 |
| 79 | 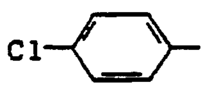 | $-NH-(CH_2)_3-CH_3$ | N | 114 |
| 80 | 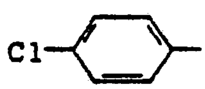 | $-NH-(CH_2)_2-CH_3$ | N | 118 |
| 81 | 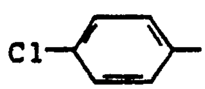 | $-NH-CH_2-CH(CH_3)_2$ | N | 140 |
| 82 | 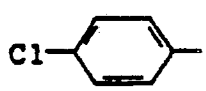 | $-NH-CH_2-$ 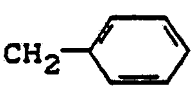 | N | 162 |

| Bsp. Nr. | Ar | R | Z | Schmelzpunkt /°C |
|---|---|---|---|---|
| 83 | 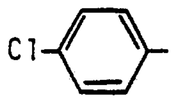 | -NH-$CH_2$- 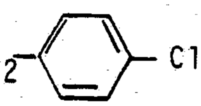 | N | 138 |
| 84 | 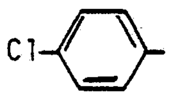 | -NH-$CH_2$-$CH_2$- 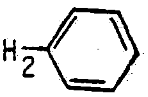 | N | 108 |
| 85 | 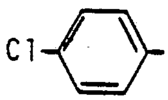 | -NH-$(CH_2)_5$-$CH_3$ | N | 84 |
| 86 | 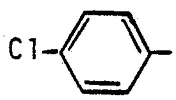 | -NH-$CH_2$- 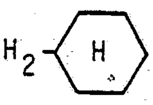 | N | 98 |
| 87 | 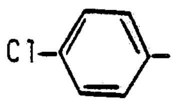 | -NH-$(CH_2)_{11}$-$CH_3$ | N | 88 |
| 88 | 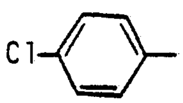 | -NH-$CH_2$-$CH_2$-$OCH_3$ | N | 106 |
| 89 | 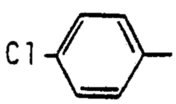 | -NH-$(CH_2)_3$-$OCH_3$ | N | 68 |
| 90 | 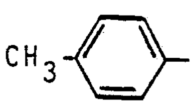 | -$CH_2$- 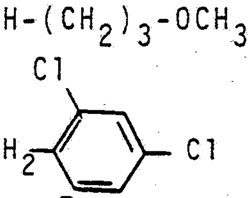 | N | 90 |
| 91 | 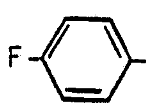 | 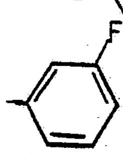 | N | 130 |
| 92 | 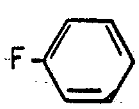 | 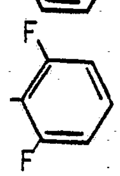 | N | 118 |

| Bsp. Nr. | Ar | R | Z | Schmelzpunkt /°C |
|---|---|---|---|---|
| 93 | 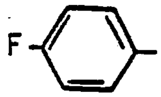 | 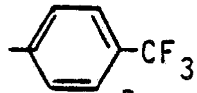 | N | 146 |
| 94 | 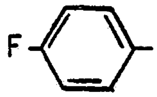 | 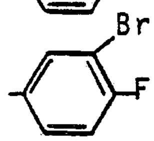 | N | 126 |
| 95 | 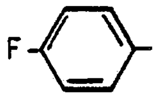 | 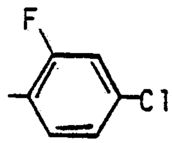 | N | 144 |
| 96 | 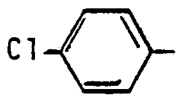 | $-CH(CH_3)_2$ | N | 140 |
| 97 | 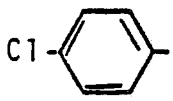 | $-C(CH_3)_3$ | N | 132 |
| 98 | 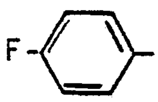 | 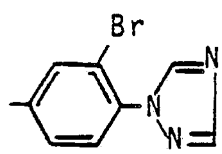 | N | 134 |
| 99 | 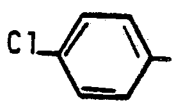 | $-(CH_2)_3-CH_3$ . | N | $n_D^{20}$ 1.5739 |
| 100 | 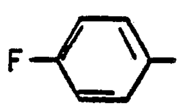 | 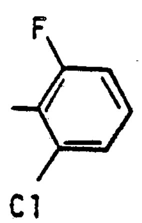 | N | $n_D^{20}$ 1.5933 |
| 101 | 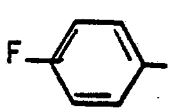 | 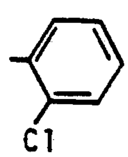 | N | 102 |

## Anwendungsbeispiele

In den folgenden Anwendungsbeispielen wurden die nachstehend aufgeführten Verbindungen als Vergleichsubstanzen eingesetzt:

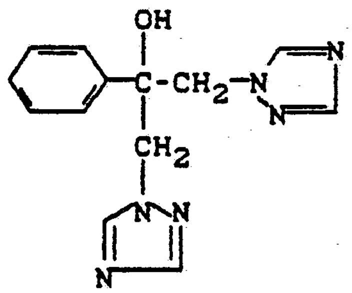

(A)

2-Phenyl-1,3-bis-(1,2,4-triazol-1-yl)-propan-2-ol

(bekannt aus EP 44 605)

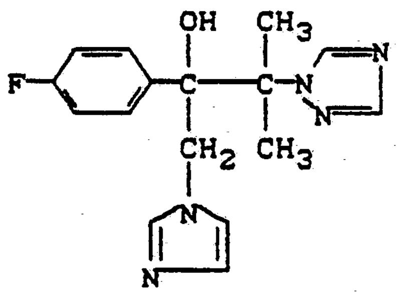

(B)

2-(4-Fluorphenyl)-1-imidazol-1-yl-3-methyl-3-(1,2,4-triazol-1-yl)-butan-2-ol

(bekannt aus EP 120 276)

Beispiel A

Antimykotische in-vitro-Wirksamkeit

Versuchsbeschreibung:

Die in-vitro-Prüfungen wurden im Reihenverdünnungstest mit Keiminokula von durchschnittlich 5 x $10^3$ bis $10^4$ Keimen/ml Substrat durchgeführt. Als Nährmedium dienten

a) für Dermatophyten und Schimmelpilze:
Sabouraud's milieu d'epreuve

b) für Hefen:
Fleischextrakt-Traubenzucker-Bouillon.

Die Bebrütungstemperatur betrug 28 °C bis 37 °C, die Bebrütungsdauer lag bei 24 bis 96 Stunden bei Hefen und 96 Stunden bei Dermatophyten und Schimmelpilzen.

In diesem Test zeigen die erfindungegemäß verwendbaren Verbindungen 1, 3, 14, 15, 18, 20, 21, 22, 23, 25, 26, 27, 28, 36, 39, 40, 41, 42, 43, 44, 45, 47, 49, 52, 59 und 62 eine bessere antimykotische Wirksamkeit als die aus dem Stand der Technik bekannten Verbindungen (A) und (B).

Tabelle A

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (A) (bekannt) 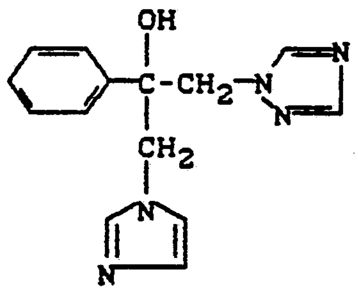 | 64 | - | 64 | >64 | >64 |
| (B) (bekannt) 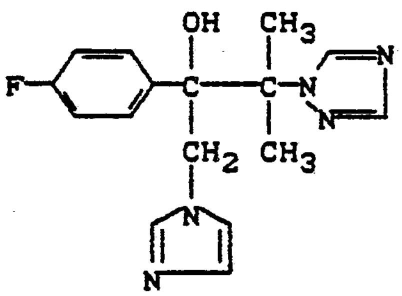 | >64 | - | >64 | >64 | >64 |
| (1) 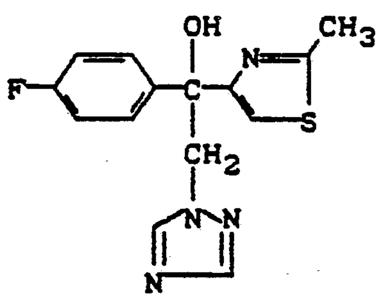 | 4 | - | 15 | >64 | 32 |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (3) 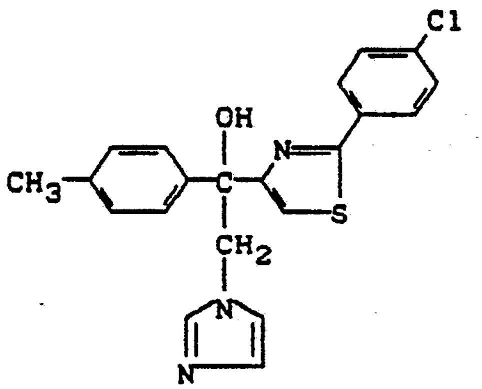 | <1 | 16 | <1 | 4 | 16 |
| (14) 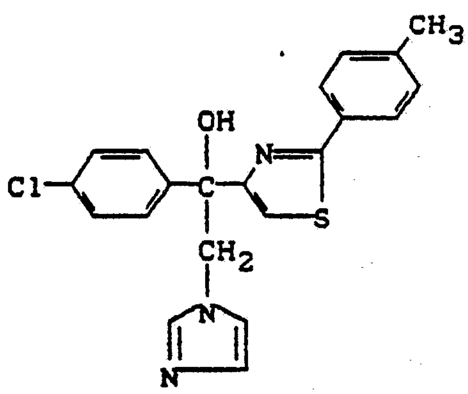 | <1 | 8 | 2 | 2 | 4 |

Tabelle A - Fortsetzung

Antimykotische in-vitro-Wirksamkeit
MHK*)-Werte in µg/ml Nährmedium

| Wirkstoff | Trichophyton mentagr. | Microsporum canis | Candida albicans | Torulopsis glabrata | Aspergillus fumigatus |
|---|---|---|---|---|---|
| (15) 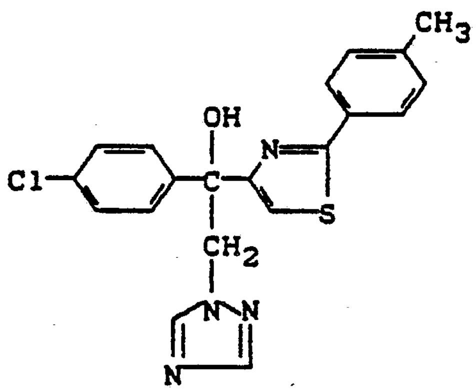 | <1 | 8 | 4 | 4 | 4 |
| (18) 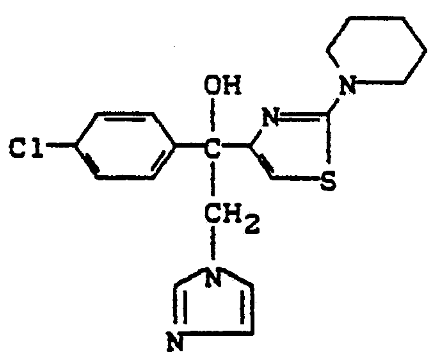 | <1 | 64 | 8 | 16 | 32 |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (20) 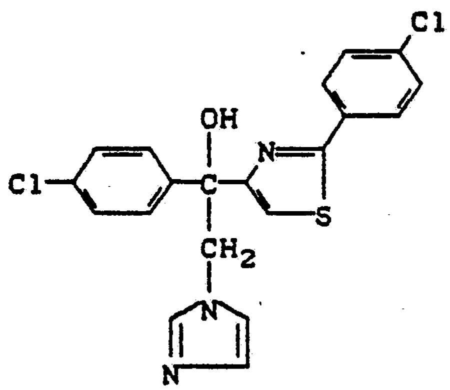 | <1 | 16 | 8 | 2 | 8 |
| (21) 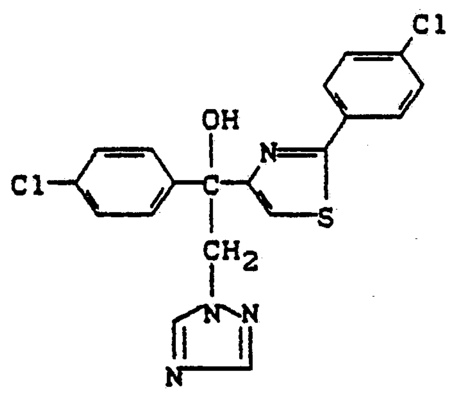 | <1 | 4 | 8 | 4 | 4 |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (22) 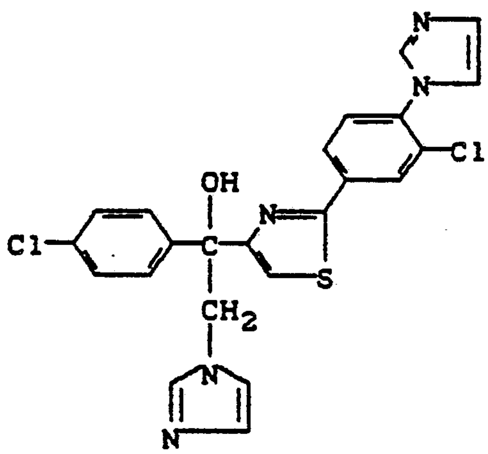 | 8 | - | <1 | 16 | 32 |
| (23) 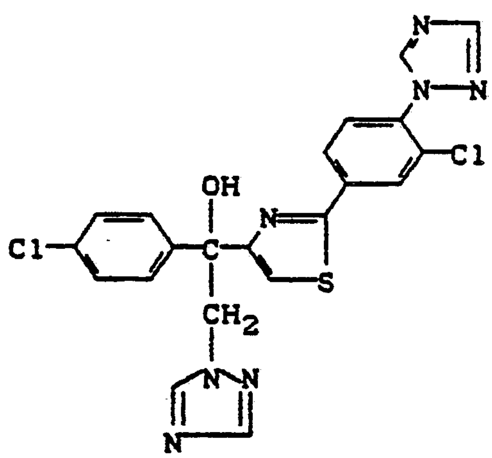 | <1 | 8 | <1 | 16 | 8 |

Tabelle A - Fortsetzung

Antimykotische in-vitro-Wirksamkeit
MHK*)-Werte in µg/ml Nährmedium

| Wirkstoff | Trichophyton mentagr. | Microsporum canis | Candida albicans | Torulopsis glabrata | Aspergillus fumigatus |
|---|---|---|---|---|---|
| (25) | <1 | 16 | <1 | >64 | 4 |
| 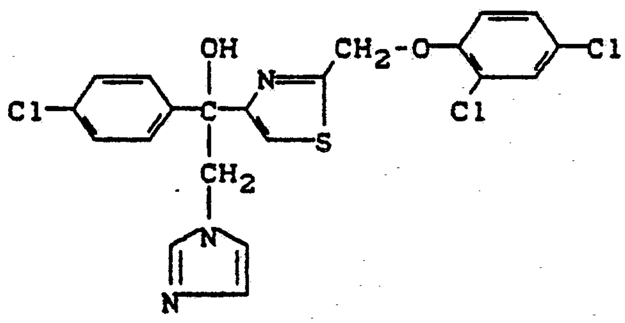 | | | | | |
| (26) | <1 | 8 | 32 | 4 | 16 |
| 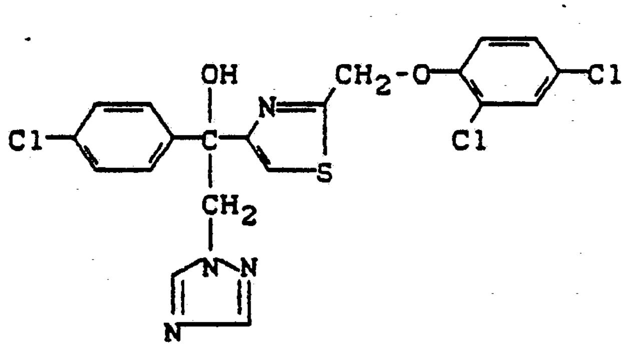 | | | | | |

Tabelle A - Fortsetzung

Antimykotische in-vitro-Wirksamkeit
MHK*)-Werte in µg/ml Nährmedium

| Wirkstoff | Trichophyton mentagr. | Microsporum canis | Candida albicans | Torulopsis glabrata | Aspergillus fumigatus |
|---|---|---|---|---|---|
| (27) 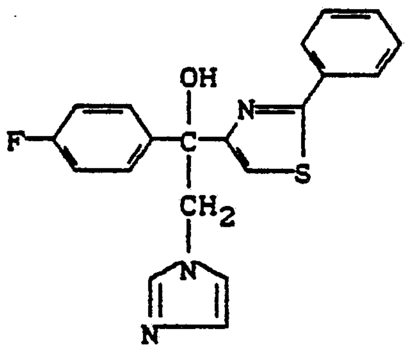 | <1 | 8 | <1 | 16 | 4 |
| (28) 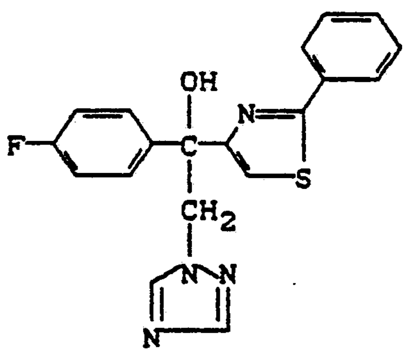 | <1 | <1 | 2 | 16 | <1 |

Tabelle A - Fortsetzung

Antimykotische in-vitro-Wirksamkeit
MHK*)-Werte in µg/ml Nährmedium

| Wirkstoff | Trichophyton mentagr. | Microsporum canis | Candida albicans | Torulopsis glabrata | Aspergillus fumigatus |
|---|---|---|---|---|---|
| (36) | <1 | 64 | 8 | 8 | 4 |
| 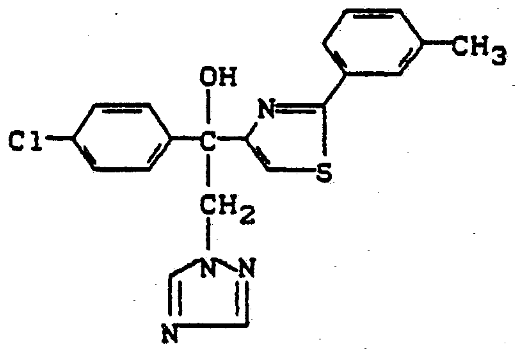 | | | | | |
| (39) | <1 | 4 | 2 | 2 | <1 |
| 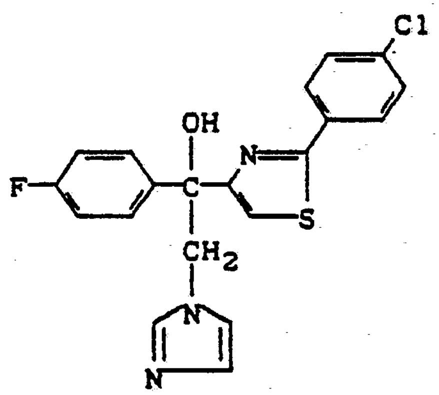 | | | | | |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (40) 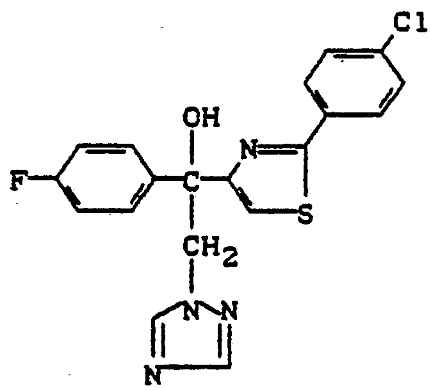 | <1 | 4 | 2 | 2 | 2 |
| (41) 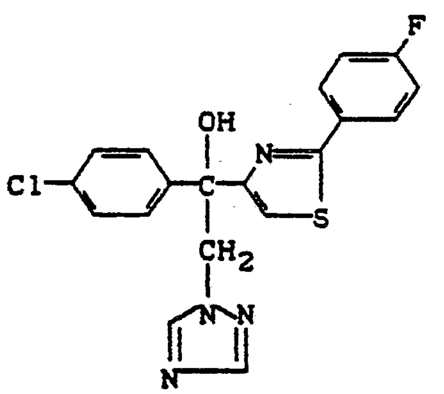 | <1 | 8 | 8 | 2 | 16 |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (42) 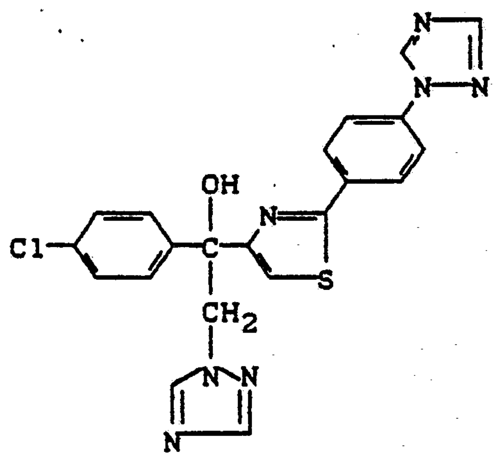 | <1 | 8 | >1 | 8 | 32 |
| (43) 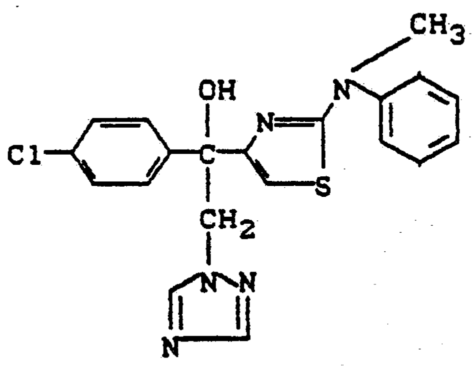 | <1 | 16 | <1 | 8 | 16 |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (44) 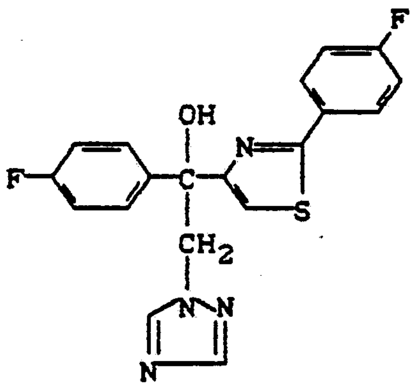 | <1 | 2 | 4 | 4 | 4 |
| (45) 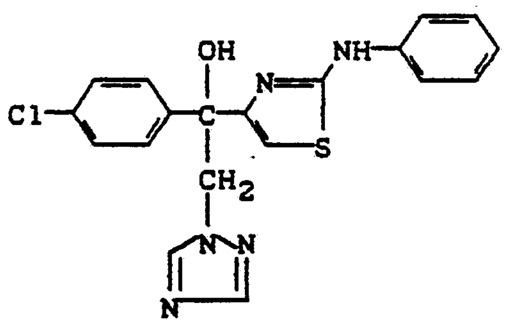 | 2 | - | 16 | 16 | 64 |

Tabelle A - Fortsetzung

Antimykotische in-vitro-Wirksamkeit
MHK*)-Werte in µg/ml Nährmedium

| Wirkstoff | Tricho-phyton mentagr. | Micro-sporum canis | Candida albi-cans | Toru-lopsis gla-brata | Asper-gillus fumi-gatus |
|---|---|---|---|---|---|
| (47) 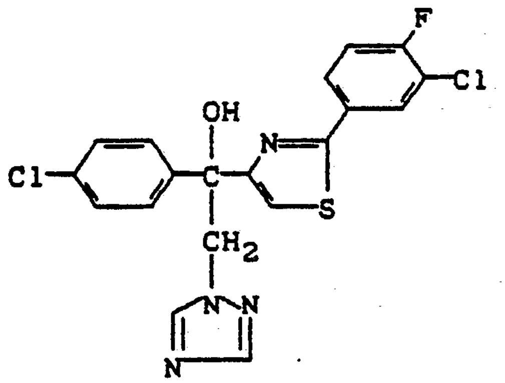 | <1 | 8 | 4 | 4 | 8 |
| (49) 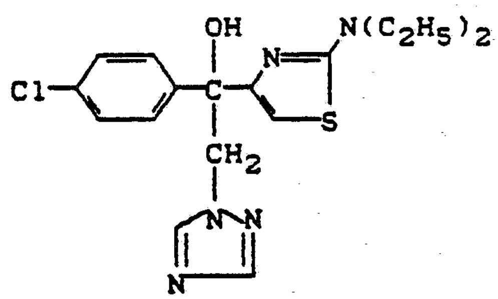 | 2 | - | 4 | 8 | 32 |

Tabelle A - Fortsetzung

Antimykotische in-vitro-Wirksamkeit
MHK*)-Werte in µg/ml Nährmedium

| Wirkstoff | Trichophyton mentagr. | Microsporum canis | Candida albicans | Torulopsis glabrata | Aspergillus fumigatus |
|---|---|---|---|---|---|
| (52) 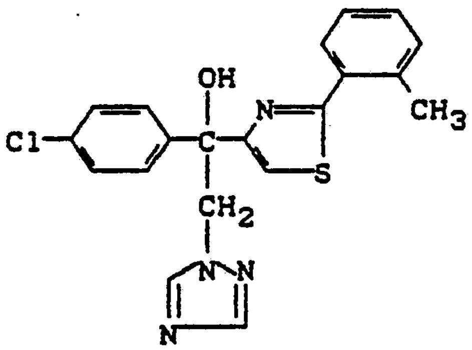 | <1 | 4 | 4 | 32 | 4 |
| (59) 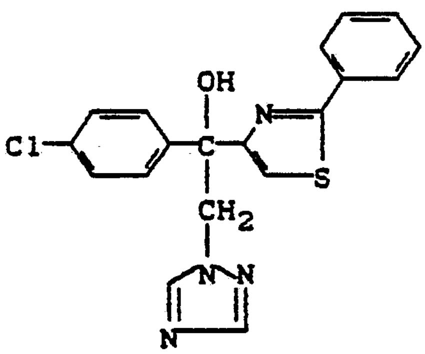 | <1 | 4 | <1 | <1 | 8 |

Tabelle A - Fortsetzung

Antimykotische in-vitro-Wirksamkeit
MHK*)-Werte in µg/ml Nährmedium

| Wirkstoff | Tricho- phyton mentagr. | Micro- sporum canis | Candida albi- cans | Toru- lopsis gla- brata | Asper- gillus fumi- gatus |
|---|---|---|---|---|---|
| (62) 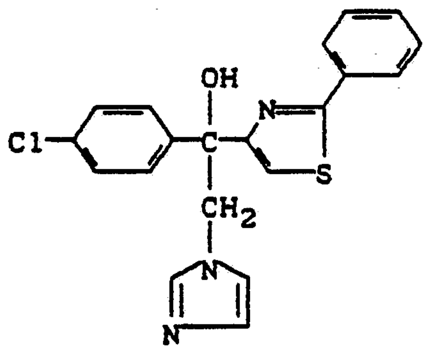 | 4 | - | <1 | 2 | 4 |

* minimale Hemmkonzentration

Beispiel B

Antimykotische in-vivo-Wirksamkeit (oral) bei Mäusecandidose

Versuchsbeschriebung :

Mäuse vom Typ SPF-$CF_1$ werden intravenös mit 1 - 2 x $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 25 - 100 mg/kg Körpergewicht der Präparate oral behandelt.

Ergebnis:

Unbehandelte Tiere sterben 3 bis 6 Tage post infektionem. Die Überlebensrate am 6.Tag post infektionem beträgt bei unbehandelten Kontrolltieren etwa 5 %.

In diesem Test zeigen z.B. die erfindungsgemäß verwendbaren Verbindungen 21, 22, 23, 40, 41, 42 und 57 eine bessere Wirkung als die aus dem Stand der Technik bekannten Verbindungen (A) und (B).

Tabelle B

Antimykotische in-vivo-Wirkung (oral) bei Mäuse-Candidose

| Wirkstoff | Wirkung |
|---|---|
| 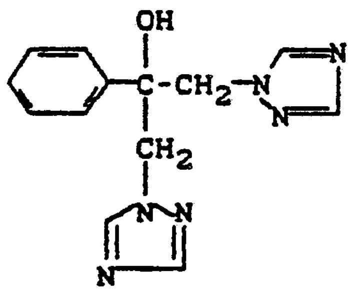 (A) (bekannt) | + |
| 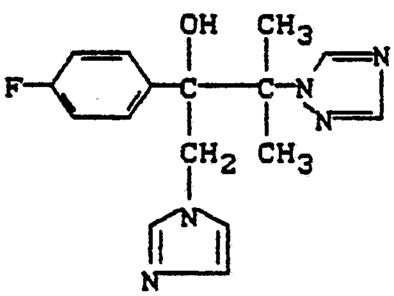 (B) (bekannt) | k.w. |
| (21) 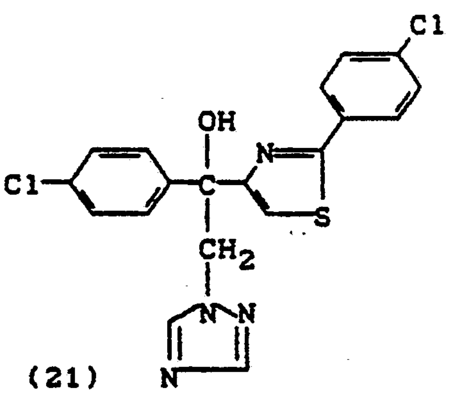 | +++ |

Tabelle B - Fortsetzung

Antimykotische in-vivo-Wirkung (oral) bei Mäuse-Candidose

| Wirkstoff | Wirkung |
|---|---|
| 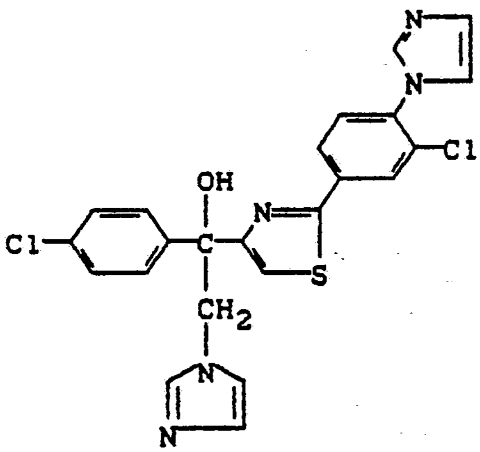 (22) | +++ |
| 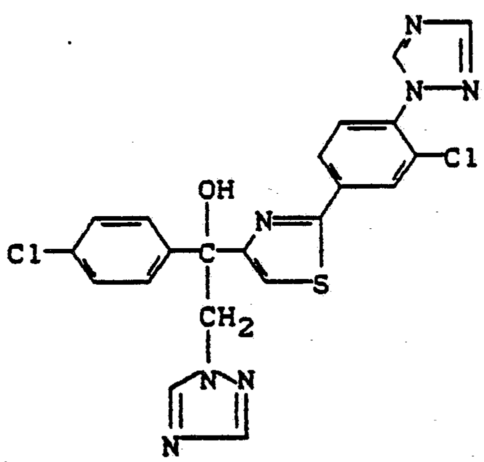 (23) | +++++ |

Tabelle B - Fortsetzung

Antimykotische in-vivo-Wirkung (oral) bei Mäuse-Candidose

| Wirkstoff | Wirkung |
|---|---|
| 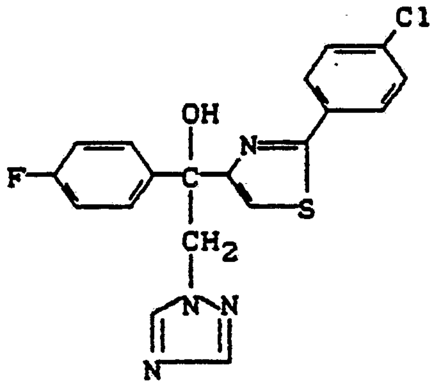 (40) | ++++ |
| 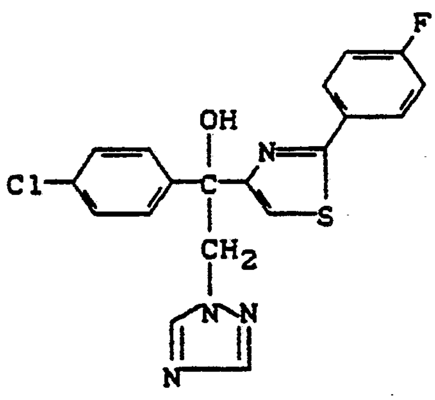 (41) | +++++ |

Tabelle B - Fortsetzung

Antimykotische in-vivo-Wirkung (oral) bei Mäuse-Candidose

| Wirkstoff | Wirkung |
|---|---|
| 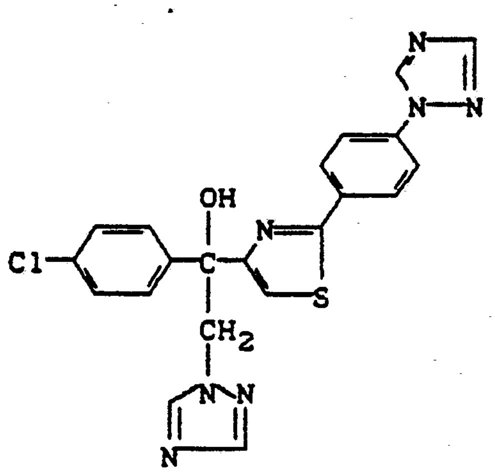 (42) | +++++ |
| 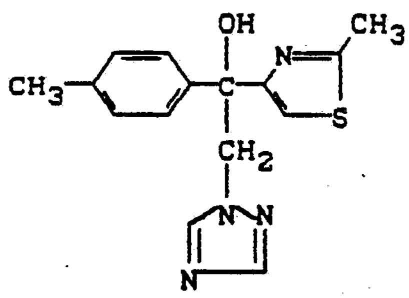 (57) | ++++ |

Zeichenerklärung:

| | | |
|---|---|---|
| +++++ | = sehr gute Wirkung | = 90 % Überlebende am 6.Tag.p.i. |
| ++++ | = gute Wirkung | = 80 % Überlebende am 6.Tag.p.i. |
| +++ | = Wirkung | = 60 % Überlebende am 6.Tag.p.i. |
| ++ | = schwache Wirkung | = 40 % Überlebende am 6.Tag.p.i. |
| + | = Spur Wirkung | = unter 40 % Überlebende am 6. Tag p.i. |
| k.W. | | = kein Unterschied zur unbehandelten Infektionskontrolle |

Beispiel C / Formulierungen

1.) Lösung:

| | | |
|---|---|---|
| Wirkstoff gemäß Formel (I): | | 10 g |
| Alkohol, rein (96 %ig) | : | 300 g |
| Isopropylmyristat | : | 526 g |
| | | 836 g |

2.) Creme:

| | | |
|---|---|---|
| Wirkstoff gemäß Formel (I) | : | 10 g |
| Arlacel 60 (Sorbitan-monostearat) | : | 20 g |
| Tween 60 (Polyoxyethylen-(20)-sorbitan-monostearat) | : | 15 g |
| Walrat, künstlich (Mischung von Estern von gesättigten Fettsäuren $C_{14}$-$C_{18}$ und Fettalkoholen $C_{14}$-$C_{18}$) | : | 30 g |
| Lanette O (Gemisch aus Cetyl-Alkohol und Stearylalkohol) | : | 100 g |
| Eutanol G (2-Octyl-dodecanol) | : | 135 g |
| Benzylalkohol | : | 10 g |
| Wasser, entmineralisiert | : | 680 g |
| | | 1000 g |

## Ansprüche

1. Azolylmethylcarbinole der allgemeinen Formel (I),

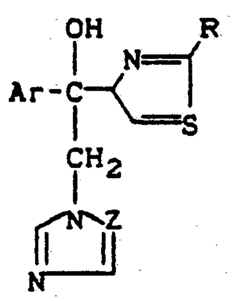

(I)

in welcher
Ar für gegebenenfalls substituiertes Aryl steht,
R für Alkyl, Alkenyl, Alkinyl, Alkoxyalkyl, für jeweils gegebenenfalls substituiertes Aralkyl, Aryloxyalkyl oder Aryl oder für einen Rest

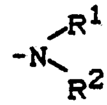

steht und
Z für Stickstoff oder für eine CH-Gruppe steht, wobei
$R^1$ für Wasserstoff, Alkyl, Alkenyl, Alkinyl oder Cycloalkyl steht und
$R^2$ für Wasserstoff, Alkyl, Alkoxyalkyl, Alkenyl, Alkinyl, Cycloalkyl, Cycloalkylalkyl, Arylalkyl oder für gegebenenfalls substituiertes Aryl steht oder
$R^1$ und $R^2$ gemeinsam mit dem Stickstoffatom, an welches sie gebunden sind, für einen gegebenenfalls substituierten Heterocyclus stehen, der gegebenenfalls weitere Heteroatome enthalten kann,
sowie deren physiologisch verträgliche Säureadditionssalze zur Bekämpfung von Krankheiten.

2. Azolylmethylcarbinole nach Anspruch 1, bei welchen
Ar für gegebenenfalls einfach oder mehrfach, gleich oder verschieden substituiertes Phenyl steht, wobei als Substituenten infrage kommen: Halogen, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor oder Brom,
R für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen oder Alkoxyalkyl mit 1 bis 6 Kohlenstoffatomen in den einzelnen Alkylteilen, für jeweils gegebenenfalls im Arylteil einfach oder mehrfach, gleich oder verschieden substituiertes, gegebenenfalls im Alkylteil geradkettiges oder verzweigtes Aralkyl, Aryloxyalkyl oder Aryl mit gegebenenfalls 1 bis 4 Kohlenstoffatomen im Alkylteil und jeweils 6 bis 10 Kohlenstoffatomen im Arylteil steht, wobei als Substituenten im Arylteil jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor oder Brom, Phenyl sowie ein gegebenenfalls einfach oder mehrfach, gleich oder verschieden durch jeweils geradkettiges oder verzweigtes Alkyl, Alkoxycarbonyl oder Alkylcarbamoyl mit jeweils 1 bis 4 Kohlenstoffatomen im Alkylteil substituierter N-verknüpfter 5-Ring- oder 6-Ring-Heterocyclus, der gegebenenfalls weitere Heteroatome wie insbesondere Stickstoff, Sauerstoff oder Schwefel enthalten kann;
außerdem für einen Rest

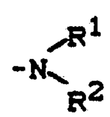

steht und
Z für Stickstoff oder für eine CH-Gruppe steht, wobei
$R^1$ für Wasserstoff, für jeweils geradkettiges oder verzweigtes Alkyl, Alkenyl oder Alkinyl mit jeweils bis zu 6 Kohlenstoffatomen oder für Cycloaklyl mit 3 bis 7 Kohlenstoffatomen steht und
$R^2$ für Wasserstoff, für jeweils geradkettiges oder verzweigtes Alkyl mit 1-12 Kohlenstoffatomen, Alkoxyalkyl mit 1-4 Kohlenstoffatomen im Alkylteil, Alkenyl oder Alkinyl mit jeweils bis zu 6 Kohlenstoffatomen, für Cycloalkyl mit 3-7 Kohlenstoffatomen, Cycloalkylalkyl mit 3-7 Kohlenstoffatomen im Cycloalkylteil und 1-4 Kohlenstoffatomen im Alkylteil, Arylalkyl mit 1-4 Kohlenstoffatomen im Alkylteil oder für gegebenenfalls einfach oder mehrfach, gleich oder verschieden substituiertes Phenyl steht, wobei als Substituenten in Frage kommen: Halogen, insbesondere Fluor, Chlor oder Brom, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, insbesondere Fluor, Chlor oder Brom oder
$R^1$ und $R^2$ gemeinsam mit dem Stickstoffatom, an welches sie gebunden sind, für einen gegebenenfalls einfach oder mehrfach, gleich oder verschieden substituierten, gesättigten Heterocyclus mit 5 bis 7 Ringgliedern stehen, der gegebenenfalls ein weiteres Heteroatom, insbesondere Stickstoff, Sauerstoff oder Schwefel enthalten kann, wobei als Substituenten jeweils infrage kommen: jeweils geradkettiges oder verzweigtes Alkyl, Hydroxyalkyl, Alkanoyl oder Alkanoyloxyalkyl mit jeweils 1 bis 4 Kohlenstoffatomen in

den einzelnen Alkylteilen,
sowie deren physiologisch verträgliche Säureadditionssalze zur Bekämpfung von Krankheiten.

3. Azolylmethylcarbinole nach Anspruch 1, bei welchen
Ar für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, n-oder i-Propoxy, Methylthio, Trifluormethyl, Trifluormethoxy oder Trifluormethylthio,
R für Methyl, Ethyl, n- oder i-Propyl, n-, i-, s- oder t-Butyl, Allyl, n- oder i-Butenyl, Propargyl, Methoxymethyl, Methoxyethyl, Ethoxymethyl, Ethoxyethyl, n- oder i-Propoxymethyl, n- oder i-Propoxyethyl oder für jeweils gegebenenfalls im Phenylteil ein- bis dreifach, gleich oder verschieden substituiertes Benzyl, Phenylethyl, Phenoxymethyl oder Phenyl steht, wobei als Substituenten jeweils infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Phenyl sowie jeweils gegebenenfalls ein- bis dreifach, gleich oder verschieden durch Methyl, Ethyl, Methoxycarbonyl, Ethoxycarbonyl, Methylcarbamoyl oder Ethylcarbamoyl substituiertes Triazolyl, Imidazolyl, Pyrazolyl, Pyrrolidinyl, Piperidinyl, Morpholinyl oder Piperazinyl;
außerdem für einen Rest

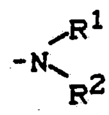

steht und
Z für Stickstoff oder für eine CH-Gruppe steht, wobei
$R^1$ für Wasserstoff, Methyl, Ethyl, n- oder i-Propyl, n-, i-, s- oder t-Butyl, Allyl, n-oder i-Butenyl, Propargyl, n- oder i-Butinyl, Cyclopentyl oder Cyclohexyl steht und
$R^2$ für Wasserstoff, Methyl, Ethyl, n- oder i-Propyl, n-, i-, s- oder t-Butyl, Allyl, n-oder i-Butenyl, Propargyl, n- oder i-Butinyl, Cyclopentyl, Cyclohexyl oder für gegebenenfalls ein- bis dreifach, gleich oder verschieden substituiertes Phenyl steht, wobei als Substituenten infrage kommen: Fluor, Chlor, Brom, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio oder
$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

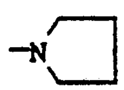 ; 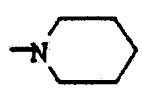 ; 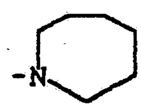 ; 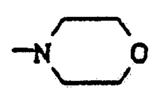 ;

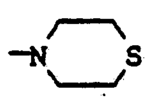 oder 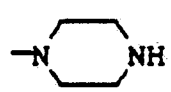

stehen, wobei
als Substituenten jeweils infrage kommen: Methyl, Ethyl, Hydroxymethyl, Acetyl, Propionyl oder Acetoxymethyl.
sowie deren physiologisch verträgliche Säureadditionssalze zur Bekämpfung von Krankheiten.

4. Azolylmethylcarbinole nach Anspruch 1, bei welchen
Ar für gegebenenfalls ein- bis zweifach, gleich oder verschieden durch Fluor, Chlor, Brom, Methyl, Methoxy, Trifluormethyl, Trifluormethoxy oder Trifluormethylthio substituiertes Phenyl steht,
R für Methyl, Ethyl, n- oder i-Propyl, Allyl, Propargyl, Methoxymethyl, für jeweils gegebenenfalls ein- bis zweifach, gleich oder verschieden substituiertes Benzyl, Phenoxymethyl oder Phenyl steht, wobei als Phenylsubstituenten jeweils infrage kommen: Fluor, Chlor, Brom, Methyl, Methoxy, Methylthio, Trifluormethyl, Trifluormethoxy, Trifluormethylthio, Phenyl sowie jeweils gegebenenfalls einfach oder zweifach, gleich oder verschieden durch Methyl, Methoxycarbonyl, Ethoxycarbonyl oder Methylcarbonyl substituiertes Triazolyl, Imidazolyl, Pyrazolyl, Piperidinyl, Morpholinyl oder Piperazinyl;
außerdem für einen Rest

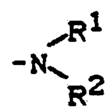

steht und
Z für Stickstoff oder für eine CH-Gruppe steht, wobei
$R^1$ für Wasserstoff, Methyl, Ethyl, n- oder i-Propyl oder Cyclohexyl steht,
$R^2$ für Wasserstoff, Methyl, Ethyl, n- oder i-Propyl, Cyclohexyl oder für gegebenenfalls einfach oder zweifach, gleich oder verschieden durch Fluor, Chlor, Brom, Methyl, Methoxy, Trifluormethyl, Trifluormethoxy oder Trifluormethylthio substituiertes Phenyl steht oder
$R^1$ und $R^2$ gemeinsam mit dem Stickstoffatom, an welches sie gebunden sind für einen gegebenenfalls ein- bis dreifach durch Methyl substituierten Heterocyclus der Formel

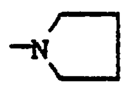 ; 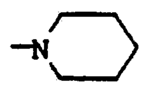 oder 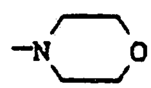

stehen oder
für einen gegebenenfalls am Stickstoff durch Methyl, Ethyl, Acetyl oder Propionyl substituierten Heterocyclus der Formel

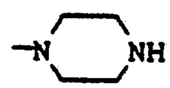

stehen,
sowie deren physiologisch verträgliche Säureadditionssalze zur Bekämpfung von Krankheiten.

5. Azolylmethylcarbinole nach Ansprüchen 1-4 zur Bekämpfung von Mykosen.
6. Arzneimittel enthaltend Azolylmethylcarbinole nach Ansprüchen 1-4.
7. Antimykotische Mittel enthaltend Azolylmethylcarbinole nach Ansprüchen 1-4.
8. Verwendung von Azolylmethylcarbinolen nach Ansprüchen 1-4 bei der Bekämpfung von Krankheiten.
9. Verwendung von Azolylmethylcarbinolen nach Ansprüchen 1-4 zur Bekämpfung von Mykosen.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 88 11 7455

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 046 337 (I.C.I.)<br>* Seiten 1-3 *<br>-- | 1-7 | C 07 D 417/06<br>A 61 K 31/425 |
| Y | EP-A-0 102 727 (PFIZER CORP.)<br>* Seiten 1-3 *<br>-- | 1-7 | |
| Y | EP-A-0 158 205 (BAYER AG)<br>* Seiten 1-3 *<br>-- | 1-7 | |
| A | EP-A-0 001 682 (I.C.I.)<br>* Seite 1 *<br>--------- | 1 | |

RECHERCHIERTE SACHGEBIETE (Int. Cl.4)

C 07 D 417/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7
Unvollständig recherchierte Patentansprüche:
Nicht recherchierte Patentansprüche: 8,9
Grund für die Beschränkung der Recherche:

Verfahren zur chirurgischen oder therapeutischen Behandlung des menschlichen oder tierischen Körpers (Siehe Art. 52(4) des Europäischen Patentübereinkommens)

| Recherchenort | Abschlußdatum der Recherche | Prüfer |
|---|---|---|
| Den Haag | 29-11-1988 | BRIGHENTI |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument